# EUROPEAN PATENT SPECIFICATION

(11) **EP 2 512 495 B1**
(45) Date of publication and mention of the grant of the patent: **09.09.2015**
(21) Application number: 11710252.5
(22) Date of filing: 06.01.2011
(51) Int. Cl.: A61K 36/28, A61P 17/10, A61P 29/00

(54) **PROCESS FOR OBTAINING A STANDARDISED EXTRACT OF QUERCETIN AN 3-O-METHYLQUERCETIN FROM FLOWERS OF MACELA (ACHYROCLINE SATUREIOIDES), AND COSMETIC AND PHARMACEUTICAL COMPOSITIONS COMPRISING SAID EXTRACT**
VERFAHREN ZUR HERSTELLUNG EINES STANDARDISIERSTES QUERCETIN UND 3-O-METHYLQUERCETIN EXTRAKTS AUS MACELA (ACHYROCLINE SATUREIOIDES) BLÜTEN, UND KOSMETISCHE UND PHARMAZEUTISCHE ZUSAMMENZETZUNGEN, DIE DEN EXTRAKT ENTHALTEN
PROCEDE POUR OBTENIR UN EXTRAIT STANDARDISE DE QUERCETINE ET DE 3-O-METHYLQUERCETINE A PARTIR D'INFLORESCENCES DE MACELA-DO-CAMPO, ET COMPOSITION COSMETIQUE, PHARMACEUTIQUE ET VETERINAIRE LE CONTENANT

(30) Priority: 15.12.2009 FR 0959012
(43) Date of publication of application: 24.10.2012
(73) Proprietor: Natura Cosméticos S.A., 06882-700 Itape Cerica da Serra SP (BR)
(72) Inventor: MOURA SA ROCHA, Vanessa de, 01420-002 - Sao Paulo - SP (BR); DELARCINA JUNIOR, Sergio, 05632-090 - Sao Paulo - SP (BR); FIGUEREDO BEDA, Débora, 05376-140 - Sao Paulo - SP (BR); LORENCINI, Marcio, 13084-551 - Campinas - SP (BR); COSTA BEBER, Tiago, 01415-010 - Sao Paulo - SP (BR); PASSERO, Alan, 06755-240 - Taboao da Serra - SP (BR)
(74) Representative: Pearson, Samuel John
(86) International application number: PCT/IB2011/000015
(87) International publication number: WO 2011/073961

(56) References cited:
- EP-A2- 1 475 097
- BR-A- 0 103 468
- US-A1- 2008 070 875
- DIAZ CLAUDIA ET AL: "Variations in the flavonoid profile and free quercetin content in different extracts of Achyrocline satureoides", ACTA FARMACEUTICA BONAERENSE, COLEGIO DE FARMACEUTICOS DE LA PROVINCIA DE BUENOS AIRES, AR, vol. 25, no. 4, 1 October 2006 (2006-10-01), pages 574-577, XP009135059, ISSN: 0326-2383
- DATABASE WPI Week 198416 Thomson Scientific, London, GB; AN 1984-098314 XP002590550, & JP 59 044313 A (YAKULT HONSHA KK) 12 March 1984 (1984-03-12)
- SIMOES C M O ET AL: "ANTIINFLAMMATORY ACTION OF ACHYROCLINE SATUREIOIDES EXTRACTS APPLIED TOPICALLY", FITOTERAPIA, IDB HOLDING, MILAN, IT, vol. 59, no. 5, 1 January 1988 (1988-01-01), pages 419-421, XP009033958, ISSN: 0367-326X
- MORQUIO A. ET AL.: "Photoprotection by topical application of Achyrocline satureioides ("Marcela")", PHYTOTHER. RES., vol. 19, 2005, pages 486-490, XP002590551, cited in the application

## Description

The present invention refers to a process to obtain an extract from inflorescences of macela-do-campo (*Achyrocline satureioides*). The present disclosure also refers to the cosmetic, pharmaceutical and veterinary compositions containing the aforesaid extract.

The present disclosure also refers to the use and method of application of the aforesaid extract of macela.

### Description of the state of the art

*Achyrocline satureioides,* also known as macela-do-campo, is a plant of the *Asteraceae* family, which is found in Argentina, Uruguay, Brazil and Paraguay and is widely used in traditional medicine. It is an annual plant, of medium height and with yellow inflorescences, commonly found in Brazil, principally in the states of the Southern region (Marques, 1995; Marques & Barros, 2000). It can be propagated from seeds (Ikuta, 1993; Marques, 1995; Marques & Barros, 2000) or by staked plant cuttings (Pardo, 1995). There is also written data about production in greenhouses (Marques & Barros, 2001) and the evaluation of the potential of the seeds for exploitation in agricultural cultivation (Ikuta, 1993; Marques, 1995; Marques & Barros, 2000).

Macela-do-campo is an annual, monoecious, branched herb, growing up to 1.5 m in height, covered in white hairs. It has alternate, complete, sessile, linear and lanceolate leaves, of up to 12 cm in length and 1.8 cm in width. It has a capitulum with two types of flowers, meeting in a corym-bose panicle; golden-yellow flowers, with one or two hermaphrodite centers, of a tubulous corolla, as well as four or five marginal flowers, which are feminine with a filiform corolla. Its fruit is achene, glabrous and brown (SIMÕES et al., 1998; MEDEIROS et al., 2005).

Pharmacological investigations of extracts and their inflorescences demonstrate sedative, analgesic, anti-inflammatory and anti-spasmodic activities (Simões, 1988; Oliveira *et al.,* 2001), anti-microbial activity against *Staphylococcus aureus* (Lemos, 2000), vasorelaxing activity (Hnatyszyn *et al.,* 2004), anti-hyperglycemic activity (Carney *et al.,* 2002) and hepatoprotective activity (Kadarian *et al.,* 2002).

It was also reported that it is commonly used for treating different pains, digestive problems (D'Ávila, 1910; Ritter *et al.,* 2002) and, more recently, for losing weight (Dickel *et al.,* 2007).

Extractive processes to obtain the extract of *Achyrocline satureioides* (macela-do-campo) and, more specifically, its use in compositions destined for the prevention and treatment of inflammatory reactions, are already known.

In this context, the Brazilian patent document PI0103468-5 can be highlighted, which discloses an extraction process of the active compounds of *Achyrocline satureioides.*

The use of the product resulting from that process has the potential to be used in the preparation of medicines for the treatment of gastrointestinal disturbances, arising from indigestion (anti-spasmodic) and for the treatment of the inflammatory conditions and topical viral infections related to herpes.

Basically, the extraction process that is the object of that Brazilian patent document includes the following stages: prepare the material that consists of dried and ground inflorescences of *Achyrocline satureioides;* place this material in contact with a macerator containing a polar organic solvent such as C₁ to C₄ alcohol or mixture with water; separate the solids present in the extraction mixture from the liquid phase; treat the solution with adjuvant agents such as colloidal silicon dioxide, for example; and dry the resultant extract.

After evaluating a document written by the same inventors (Bassani et al. 1997), and prior to that patent document, it was learned that the process consists of a maceration that uses a solvent solution in the proportion of 7.5% (p/v), or rather, 7.5g of the plant for every 100 ml of solvent solution. Furthermore, for each phase of the extraction process, object of that state of the art document, the mixture can, preferentially, be ethanol/water in the proportion of 80% ethanol and 20% water or in increasing concentrations of ethanol up to 100% ethanol. In this context, it should be observed that the above mentioned Brazilian state of the art document describes a process that requires a long time, due to the use of maceration, whose extraction occurs at an ambient temperature and can take from 24 to 160 hours, according to the document. Furthermore, the process is of low efficiency for the extraction of the compounds of interest, namely, quercetin and 3-O-methylquercetin, because the dried extract contains those compounds in concentrations of, at the most, 0.9391% and 0.7294%, respectively. It is also known that in this prior art patent document, the 3-O-methylquercetin is dosed concomitantly with luteolin, resulting in the content of the 3-O-methylquercetin being even less than 0.7294%.

In relation to the use of extracts from *Achyrocline satureioides* in cosmetic and pharmaceutical products, the Japanese document JP10226619, which discloses a skin preparation for external use that presents an anti-oxidant action in order to prevent the oxidation of the lipidic components using the extract of a plant of the genus *Achyrocline,* such as macela-do-campo *(Achyrocline satureioides),* for example, is highlighted. That Japanese document discloses a process that involves the flowers, stem, roots, leaves and seeds of the species belonging to the genus *Achyrocline.* However, it is known to a person skilled in the art that the substances of interest for the benefits of our patent, namely the quercetin and the 3-O-methylquercetin, are concentrated in the inflorescences and are not found in suitable quantities in the other organs, such as the leaves, stems and roots for the production of a dried extract concentrated in these substances.

The document JP 59044313 discloses an anti-bacterial composition that is useful as an effective cosmetic in the treatment of acne, etc, using quercetin as an active component and rutin and isorhamnetin or 3-O-rutinoside as essential and auxiliary components. The anti-bacterial activity of the composition is elevated in a weakly acidic pH of approximately 5.0-5.5 and the composition is suitable for use in the treatment of acne against *Propionibacterium acnes.* The concentration of the quercetin is preferentially 0.2-0.6 % in weight.

The North American document US 2004/0170581 also discloses cosmetic preparations that contain an effective quantity of at least one ester derived from caffeic acid. One of the activities attributed to these esters is based on its anti-inflammatory potential.

This North American document also describes the sources to obtain the aforementioned caffeic acid. The anti-inflammatory mechanism of the esters of caffeic acid described by this document is based on its inhibitory action on the release of the pro-inflammatory mediators, by the mast cells, eosinophils and basophils.

The North American document US 7,192,611 discloses a method of treatment of osteoarthritis that comprises of the administration of a composition based on flavonoids with an unsubstituted B ring and, optionally, a physiologically acceptable vehicle. The species from the genus *Achyrocline* are mentioned among the sources claimed as options for the extraction of the aforesaid flavonoids.

The North American document US 2008/0070875 discloses a composition for the treatment of acne that includes an anti-microbial / molecule of anti-inflammatory polyphenol(s), including quercetin and rosmarinic acid in combination with the salicylic acid and/or salicylate salts in a cosmetic vehicle. It also refers to a method for the treatment of acne by topically administering a composition in a therapeutically effective quantity in order to reduce the redness and spots associated with acne. That document does not mention macela-do-campo as a source of active ingredients to be used in the treatment of acne.

Accordingly, that North American document is directly related to the anti-inflammatory potential (inhibition of COX-2) of extracts derived from the species belonging to the genus *Achyrocline.*

The North American document US 2004/0220119 describes a method of the treatment of diseases mediated by the action of COX and LOX. This method is based on the local application of a composition containing flavonoids with an unsubstituted B ring and, at least, one flavan. One of the options claimed as a source to obtain the flavonoids with an unsubstituted B ring are the plant species belonging to the genus *Achyrocline.*

As is known to a person skilled in the art, the enzymatic chains COX and LOX are directly related to the inflammation mechanisms. Therefore, this document makes reference to the anti-inflammatory potential of the extracts from the species belonging to the genus *Achyrocline.*

In addition, those North American documents are not directly related to the cosmetic environment and, therefore, the bases of the compositions of topical application capable of stabilizing the flavonoids with an unsubstituted B ring are not described in detail.

From the following description of the present invention, it can be concluded that no document of the state of the art presents, as an object, a process of multiple alcoholic and/or hydro-alcoholic extractions of the inflorescences of *Achyrocline satureioides* (macela-do-campo), resulting in an extract containing, concurrently, quercetin and 3-O-methylquercetin in an elevated concentration. Furthermore, the extract of macela-do-campo resulting from the process of the present invention presents anti-inflammatory, anti-oxidant and anti-microbial activities.

Also, cosmetic, pharmaceutical and veterinary compositions will be described containing the aforesaid extract of macela-do-campo, used for the cosmetic and pharmaceutical prevention and treatment of the pathologies arising from inflammatory processes, microbial infections and cellular lesions by oxidative processes.

### Objectives of the invention

The present invention has an objective to provide a process of extraction of inflorescences of *Achyrocline satureioides* (macela-do-campo) that results in an extract containing elevated concentrations of quercetin and 3-O-methylquercetin presenting a high yield and a low use of ethanol.

### Summary of the invention

The present invention refers a process for obtaining a standardized extract of quercetin and 3-O-methylquercetin from inflorescences of *Achyrocline* comprising of the following steps:
A) grinding the inflorescences of *Achyrocline satureioides* to obtain a material of ground plant;
B) submitting the ground plant to at least four sequential stages of hydro-alcoholic extraction at a temperature from 60°C to 80°C, for 3 to 4 hours for each stage, in order to obtain 4 intermediary hydro-alcoholic extracts;
C) combining the 4 intermediary hydro-alcoholic extracts;
D) concentrating the intermediary hydro-alcoholic extracts mixture; in order to obtain up to a maximum of 20% of the initial mass of the intermediary hydro-alcoholic extracts;
E) drying the material obtained in (D).

Also disclosed herein is a cosmetic composition and a pharmaceutical composition comprising, in a physiologically acceptable vehicle, an extract of inflorescence of macela-do-campo obtained from the process of extraction of inflorescences of *Achyrocline satureioides* described above.

### Brief description of the figures

Figure 1 illustrates a scheme of a preferred embodiment of the process of extraction, object of the present invention;
Figure 2 illustrates the result of the trial of the phytochemical profile of extracts obtained from the process, object of the present invention;
Figure 3 illustrates the result of the trial of the High Performance Thin Layer Chromatography performed with three examples of extracts obtained by the process, object of the present invention;
Figure 4 illustrates the result of the test of phototoxicity, referring to the extract of macela-do-campo obtained by the process, object of the present invention;
Figure 5 illustrates the result of the test of cytotoxicity, referring to the extract of macela-do-campo obtained by the process, object of the present invention;
Figure 6 illustrates, on the left, the separation of the compounds of the extract of inflorescence of macela-do-campo with a solvent system of chloroform/ ethyl acetate /methanol/water (60:32:12:8) and revealed with NP/PEG. On the right the inhibition halos formed after the incubation for 72 hours with *P. acnes* ATCC 6538 and revealed with tetrazolium chloride (TTC) is shown;
Figure 7 illustrates the result of the test of activity of the extract of inflorescence of macela-do-campo in a of fibroblast model with induction by LPS;
Figure 8 illustrates the result of the test of activity of the extract of inflorescence of macela-do-campo in a of fibroblast model with induction by UVB;
Figure 9 illustrates the result of the trial of anti-inflammatory activity of the extract of inflorescence of macela-do-campo in a model of human keratinocytes (HaCaT) strain by the induction of production of cytokine by *Propioniumbacferium acnes* inactivated by temperature (80°C, 30 min);
Figure 10 illustrates the result of the test of anti-oxidant activity of the extract of macela obtained from the process, object of the present invention.
Figure 11 illustrates the result of the test of Minimum Inhibitory Concentration (MIC) for two batches and the carrier.
Figure 12 illustrates the result of the test of Minimum Inhibitory Concentration (MIC) for a third batch, quercetin and the carrier.

### Detailed description of the invention

The present invention refers to a process for obtaining a standardized extract of quercetin and 3-O-methylquercetin from inflorescences of *Achyrocline satureioides* comprising of the following steps:
- grinding the inflorescences of *Achyrocline satureioides* to obtain a material of ground plant;
- submitting the ground plant to at least four sequential stages of ethanol or hydro-alcoholic extraction (at least 50% of solvent being alcohol) under heating of 60°C to 80°C, for 3 to 4 hours for each stage, in order to obtain 4 intermediary hydro-alcoholic extracts;
- combining the 4 intermediary hydro-alcoholic extracts;
- concentrating the intermediary hydro-alcoholic extracts mixture up to a maximum of 20% of the initial mass of the intermediary hydro-alcoholic extracts;
- drying the resulting material.

Preferably the sequential steps of hydro-alcoholic extractions are carried out under reflux, for 12 hours and comprise the following stages:
i) extracting an intermediary extract with a hydro-alcoholic solution 1:30 (m/m of ground plant material/extracting solution) for a period of 3 to 4 hours, preferably for at least 3 hours, at a temperature in the range of 60 to 80°C, preferably at 80°C, in order to obtain a first extracted solution;
ii) re-extracting the hydro-alcoholic extract of (i) with a hydro-alcoholic solution in the proportion of 1:20 (m/m of plant drug/extracting solution) for a period of 3 hours at a temperature of 80°C thus obtaining a second extracted solution;
iii) combining the first and the second extracted solutions in order to obtain a third extracted solution;
iv) extracting a fresh batch of ground plant with said third extracted solution in the proportion of 1:30 (m/m of ground plant ingredient/extracted solution of stages 1 and 2) for a period of 3 hours at a temperature of 80°C ;
v) re-extracting the hydro-alcoholic extract from stage (iv) with a hydro-alcoholic solution in the proportion of 1:16 (m/m of hydro-alcoholic extract /extracting solution) for 3 hours at 80°C in order to obtain a final extracted solution.

The process of extraction, object of the present invention, is highly efficient (up to 64.62% of yield in relation to quercetin according to the preferential embodiment) with a reduced consumption of solvent. For example, 546,875 liters of ethanol were used for 28.13 kg of inflorescences of macela-do-campo, that is, 19.44 liters of ethanol for each kg of inflorescences of macela-do-campo.

It is a clean process (without undesirable byproducts or the use of solvents harmful to the environment), presenting an optimized result in relation to the teachings of prior art. It provides an extract with 3 times more quercetin than that described in PI0103468-5, using less ethanol and also comprising of O-3-methyl-quercetin.

It presents an ideal constitution for use as an anti-inflammatory, anti-oxidant and anti-microbial, as will detailed below.

The preferable proportions of ethanol and water used in the stages of extraction are 1:1 m/m ethanol:water or 1.25:1 v/v ethanol:water, and the amount of water may be reduced until only ethanol is used.

According to the process of the present invention, the chemical compounds quercetin (CAS 117-39-5) and 3-O-methylquercetin (CAS 1486-70-0) are maintained and concentrated in the final resulting extract.

Data is not found in the technical literature that describes the presence of these two components, concomitantly, and in similar proportions to those described by this process, derived from the inflorescences of macela-do-campo. On evaluation of the process object of the present invention, it can be perceived that one of the advantages of the aforesaid process is its low cost because the solvents are readily available and well known. The equipment is conventional and has a reduced environmental impact due to the lower quantity of organic residues eliminated after the process. This is because the evaporated alcohol can be re-used for a new extraction of inflorescences of macela-do-campo.

In a preferred embodiment of the present invention, the process to obtain the extract of macela-do-campo comprises of the following steps:
i) Grinding the inflorescences of macela, in order to obtain the ground plant drug in a knife grinder;
ii) Mixing the ground plant drug with an extracting hydro-alcoholic solution in the proportion of 1:1 (m/m), using ethanol 96°GL as alcohol, in the initial proportion of 1 part plant ingredient to 30 parts of hydro-alcoholic solution, based on the mass of the materials. Performing the extraction of the compounds for a period of at least 3 hours at a temperature of 80°C in a closed reactor;
iii) Filtering the mixture obtained in ii) in order to obtain a first intermediary hydro-alcoholic extract and a first residue consisting of the components of the non-extracted plant drug;
iv) Mixing the first residue consisting of the components of the non-extracted plant drug with a fresh extracting hydro-alcoholic solution, constituted of a mixture of ethanol 96°GL and water in the proportion 1:1 (m/m). The mass of extracting hydro-alcoholic solution to be added is 2/3 of the mass of the first extracting hydro-alcoholic solution for a period of 3 hours at a temperature of 80°C in a closed reactor;
v) Filtering the mixture obtained in iv) in order to obtain a second intermediary hydro-alcoholic extract and a second residue constituted of the components of the non-extracted plant drug;
vi) Grinding a second quantity of plant drug, obtaining a second quantity of ground plant drug;
vii) Mixing the first and second intermediary hydro-alcoholic extracts to the second quantity of ground plant drug obtained in vi) in the proportion of 1:30 (m/m of plant drug/extracting hydro-alcoholic solution) for a period of 3 hours at a temperature of 80°C (range: 60°C to 80°C) in a closed reactor;
viii) Filtering the mixture obtained in vii) in order to obtain a third intermediary hydro-alcoholic extract and a third residue constituted of the components of the non-extracted plant drug;
ix) Mixing the third residue constituted of the components of the non-extracted plant drug with a new hydro-alcoholic solution, constituted of a mixture of ethanol 96°GL and water in the proportion 1:1 (m/m). The mass of the new extracting hydro-alcoholic solution to be added is 11/20 of the mass of the third intermediary hydro-alcoholic extract for a period of 3 hours at a temperature of 80°C in a closed reactor;
x) Filtering the mixture obtained in ix) in order to obtain a fourth intermediary hydro-alcoholic extract and a fourth residue constituted of the components of the non-extracted plant drug;
xi) Evaporate the ethanol from the mixture obtained in xi) by vacuum assisted evaporation (temperature of 60° C, vacuum of 700 mm Hg) until the mass corresponds to approximately 25% of the mass of the mixture obtained in x);
xii) Mixing the concentrate of xi) with an appropriate vehicle for the carrying out of the drying by spray dryer, preferably maltodextrin in the maximum proportion in mass of 50% of the solids expected in the product when dry.
xiii) Spraying the suspension obtained in xii) in a spray dryer with an entrance temperature of 185°C and an exit temperature of 85°C.

In one of the preferential embodiments of the present invention, the final stage of the concentration is performed in a vacuum concentrator at a temperature of 60°C and a vacuum of 600 mmHg, wherein the aforesaid final hydro-alcoholic extract is concentrated approximately 4 times. After the concentration step, a polysaccharide is added to the solution respecting a proportion of 50% in mass regarding the estimate of dry mass present in the solution, this preferentially being genetically non-modified maltodextrin. Other materials that can also be added at this stage are silicon dioxide, amide and cyclodextrin. This mixture is submitted to drying by spray dryer (entrance temperature of 180/185°C and exit temperature of 80/85°C under agitation) in order to obtain a dried extract.

As an example, the equipment that can be used in the process, object of the present invention, is:
- **Grinder:** hammer mill with sieves openings of 5 and 10 mm;
- **Reactor :** capacity of 750 liters;
- **Concentrator:** concentrator by convectional evaporation, productive capacity of 50 - 200 kg/h, installed power of 7.5 kW;
- **Spray dryer:** atomizer dryer, concurrent flow with a rotating disc, evaporation capacity of water of 15 - 25 l/hour.

The content of evaporated ethanol in the final concentration stage will be determined by alcometers and re-used in a new extractive process of macela-do-campo.

It is necessary to operate with at least 4 stages of distinct and sequential extractions in order to obtain an extract of macela-do-campo with the concentrated compounds of interest. If one simple stage of extraction was employed, the required concentration would not be achieved. Furthermore, proportionally, the volume of solvent used can be much less than that used in a single stage because the solvent can be re-used in successive stages.

The use of other solvents or the performance of other processes to obtain the extract of macela can result in the loss of at least one of the chemical components of interest: quercetin and/or 3-O-methyl-quercetin.

During the process of the present invention, the solvents used for the extraction are basically ethanol and water, in the proportion of 15.55 kg water and 15.55 kg of ethanol for each 1 kg of plant drug to be submitted to the process of extraction. For each phase of extraction of the process according to the present invention, the mixture can, preferentially, be ethanol/water (1:1 m/m) up to entirely ethanol.

With the performance of all the abovementioned stages, an extract of macela is obtained which is standardized in quercetin and 3-O-methylquercetin, obtained by an extractive process with a yield of up to 67.4% in relation to the mass of quercetin. The concentrations of quercetin and 3-O-methylquercetin are up to 301% and 262%, respectively, in relation to the mass of quercetin (CAS 117-39-5) and 3-O-methylquercetin (CAS 1486-70-0) present in the inflorescences. The actual content obtained by the process was up to 3.01% for the quercetin and up to 2.62% for the 3-O-methylquercetin. The dried extract was obtained from a plant drug with a content of 1.00% of quercetin in a dry base.

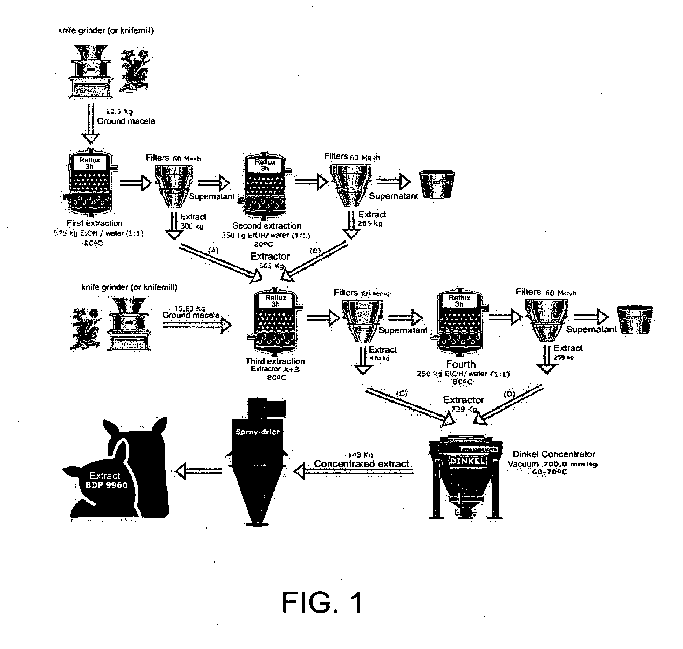

Figure 1 illustrates a scheme of an example of a preferential embodiment of the process of extraction, object of the present invention.

The dried extract of inflorescences of *Achyrocline satureioides* (macela-do-campo) resulting from the process according to the present invention, is constituted, basically, of high concentrations of the flavonoids: quercetin and 3-O-methylquercetin. This standardized dried extract presents anti-inflammatory, antioxidant and anti-microbial activities.

The factors that should be controlled in this process, in particular, are:
- the proportion of the composition of the extractive hydro-alcoholic mixture should be one part of ethanol for one part of water, expressed by mass;
- the temperature used in the extraction steps should be between 60°C and 80°C;
- the time of the extraction should be between 3 hours and 4 hours;
- the temperature of drying when performed in a spray dryer should be between 180°C and 185°C for the entrance of the material and between 80°C and 85°C for the exit temperature of the material;
- the sequence of the extraction stages and the amount supplied to the equipment in these stages should be respected.

The perceived advantages of employing the process according to the present invention, are highlighted as follows:
✔ the process to obtain an extract of macela is highly efficient: 60% efficiency of yield in relation to the mass of quercetin (CAS 117-39-5);
✔ the solvents used in the extraction steps are low cost and reusable. In addition, ethanol is a naturally renewable source. It is a clean process with an optimized result;
✔ the extract contains quercetin (CAS 117-39-5) and 3-O-methylquercetin (CAS 1486-70-0) in high concentrations. These two chemical compounds are not found in these concentrations in extracts obtained by only a single stage extraction. Also, the single stage process has a concentration of quercetin 3 times less than that obtained in the process of the present invention;
✔ the obtained extract has various activities and, therefore, can be used for anti-inflammatory, anti-acne, anti-microbial and antioxidant products, for the treatment of erectile dysfunction and for the pharmacological treatment of bronchial asthma;
✔ the extract can partially or totally substitute an antioxidant complex because it operates as an antioxidant in the formula;
✔ the use of the extract increases the plant content index of cosmetic formulas;
✔ the obtained extract is considered to be a product of plant origin and natural;
✔ the obtainment of the aforesaid extract has a good cost benefit relationship for the classes of pharmaceutical, cosmetic and veterinary products that it is destined for;
✔ the extract does not affect the color, smell and feel of the product.

### Example of the process of preparation of the extract of macela

The following example is a preferential embodiment of the process of preparation of the extract of macela of the present invention and should not be interpreted as a limitation to the aforesaid invention. In this context, it should be understood that the scope of the present invention has other possible embodiments and is limited only by the content of the attached claims, including all the possible equivalents therein.
i) Grind the inflorescences of macela, in order to obtain the ground plant drug in a knife grinder;
ii) Mix the ground plant ingredient with an extracting hydro-alcoholic solution in the proportion of 1:1 (m/m), using ethanol 96°GL as alcohol, in the initial proportion of 1 part plant drug to 30 parts of hydro-alcoholic solution, by mass of the materials. Perform the extraction of the compounds for a period of 4 hours at a temperature of 80°C in a closed reactor;
iii) Filter the mixture obtained in ii) in a filter with a porosity specification of 60 mesh, in order to obtain a first intermediary hydro-alcoholic extract and a first residue constituted of the components of the non-extracted plant drug;
iv) Mix the first residue constituted of the components of the non-extracted plant drug with a fresh extracting hydro-alcoholic solution, constituted of a mixture of ethanol 96°GL and water in the proportion 1:1 (m/m). The mass of extracting hydro-alcoholic solution to be added should be a maximum 2/3 of the mass of the first extracting hydro-alcoholic solution for a period of 4 hours at a temperature of 80°C in a closed reactor;
v) Filter the mixture obtained in iv) in a filter with a porosity specification of 60 mesh, in order to obtain a second intermediary hydro-alcoholic extract and a second residue constituted of the components of the non-extracted plant drug;
vi) Grind a second quantity of plant drug in a knife grinder, obtaining a second quantity of ground plant drug;
vii) Mix the first and second intermediary hydro-alcoholic extracts to the second quantity of ground plant drug obtained in vi) in the proportion of 1:30 (m/m of plant drug/extracting hydro-alcoholic solution) for a period of 4 hours at a temperature of 80°C in a closed reactor;
viii) Filter the mixture obtained in vii) in a filter with a porosity specification of 60 mesh, in order to obtain a third intermediary hydro-alcoholic extract and a third residue constituted of the components of the non-extracted plant drug;
ix) Mix the third residue constituted of the components of the non-extracted plant drug with a fresh hydro-alcoholic solution, constituted of a mixture of ethanol 96°GL and water in the proportion 1:1 (m/m). The mass to be added of the fresh extracting hydro-alcoholic solution is 11/20 of the mass of the third intermediary hydro-alcoholic extract for a period of 4 hours at a temperature of 80°C in a closed reactor;
x) Filter the mixture obtained in ix) in a filter with a porosity specification of 60 mesh, in order to obtain a fourth intermediary hydro-alcoholic extract and a fourth residue constituted of the components of the non-extracted plant drug;
xi) Evaporate the ethanol from the mixture obtained in xi) by vacuum assisted evaporation (temperature from 40° C to 60° C, vacuum of 600 to 700 mm Hg) until the mass corresponds to approximately 25% of the mass of the mixture obtained in x);
xii) Mix the concentrate obtained in xi) with an appropriate vehicle (carrier) for the carrying out of the drying by spray dryer, this being maltodextrin (or optionally silicon dioxide, amide or cyclodextrin) in the maximum proportion in mass of 50% of the solids expected in the product when dry.
xiii) Atomize the suspension obtained in xii) in a spray dryer with an entrance temperature of 180 - 185°C and an exit temperature of 80 to 85°C.

### TRIALS AND TESTS

### I. Trial by High Performance Liquid Chromatography (HPLC)

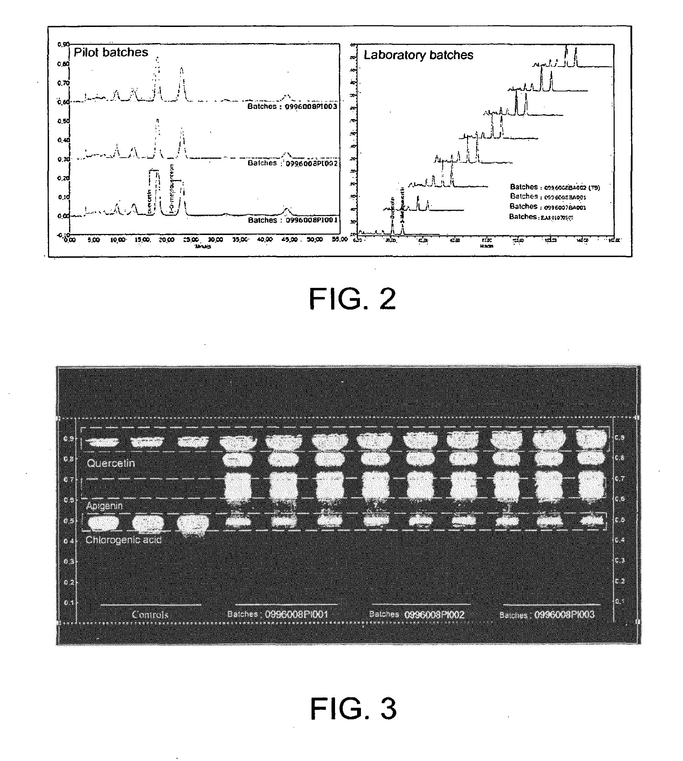

The result of the trial of comparative phytochemical profile by HPLC among the three batches obtained from the process of extraction described in the above example is illustrated in figure 2.

Table 1 below shows the content of quercetin and 3-O-methylquercetin present in the aforesaid batches of extract of inflorescences of macela-do-campo.

**Table 1**

| Sample | Compound | Average Content | Standard Deviation | Variation Coefficient |
|---|---|---|---|---|
| Batch 1 | quercetin | 2.95% | 0.01% | 0.21% |
| | 3-O-methylquercetin | 2.62% | 0.01% | 0.47% |
| Batch 2 | quercetin | 2.60% | 0.01% | 0.21% |
| | 3-O-methylquercetin | 2.30% | 0.01% | 0.42% |
| Batch 3 | quercetin | 3.01% | 0.02% | 0.72% |
| | 3-O-methylquercetin | 2.60% | 0.01% | 0.44% |

The average content was determined by the quantitative analytical method in High Performance Liquid Chromatography (HPLC) for samples in triplicate by two consecutive injections of each aliquot. The analysis of the content of quercetin (CAS 117-39-5) and 3-O-methylquercetin (CAS 1486-70-0) was used to verify the reproducibility of the process of manufacture of the three batches. In summary, aliquots were taken from the 3 batches reaching an average between 2.6% and 3.0% of quercetin and 2.3% to 2.6% of O-methylquercetin. The samples were diluted in methanol and subjected to analysis by HPLC. Conditions of the chromatographic system:
Column: Merck Lichrospher 100RP18e (4.6 x 250mm . 5um)
System: Isocratic
Movable Phase: phosphoric acid 0.5%: methanol (1:1/ v:v)
Temperature: 25°C
Flow: 0.6ml/min
Wavelength: 370nm
Volume of injection: 20µl
Run time: 55 minutes
Standard : di-hydrated quercetin (CAS 117-39-5)
Note:: the concentration of 3-O-methylquercetin (CAS 1486-70-0) was dosed as quercetin (CAS 117-39-5) because it has the same chromophoric group.

### II. Trial by High Performance Thin Layer Chromatography (HPTLC)

Figure 3 illustrates the phytochemical evaluation by HPTLC that was performed to identify the other compounds that were not observed in the HPLC. The trial demonstrated that the extraction of other compounds was also reproducible in the three batches. Other chemical compounds were identified, namely, apigenin and chlorogenic acid. The screening using techniques of thin layer chromatography allowed the comparison of the retention times of standards of reference and the coloration obtained after derivation with specific reagents. For the evaluation of the identified substances, the chromatographic plates were derived with the reagent NP/PEG and evaluated under UV light at 365nm. The reagent of NP/PEG consists of a mixture 1:1 of a solution of 5% polyethyleneglycol 4000 in ethanol and a solution of 1% diphenyl borate of aminoethanol in methanol.

### III. Tests of Cytotoxicity and Phototoxicity

The phototoxicity and cytotoxicity were evaluated for one of the batches of the extract of macela produced in accordance with the process of the above example.

The study of phototoxicity was conducted with the objective of evaluating the phototoxic potential in fibroblasts of permanent strain 3T3 in the presence and absence of UVA light.

The study of cytotoxicity was conducted with the objective of evaluating the cytotoxic potential of a substance by means of *in vitro* methodology, which evaluates the potential of cytotoxicity of the test substance in fibroblasts of the permanent strain 3T3 in different concentrations.

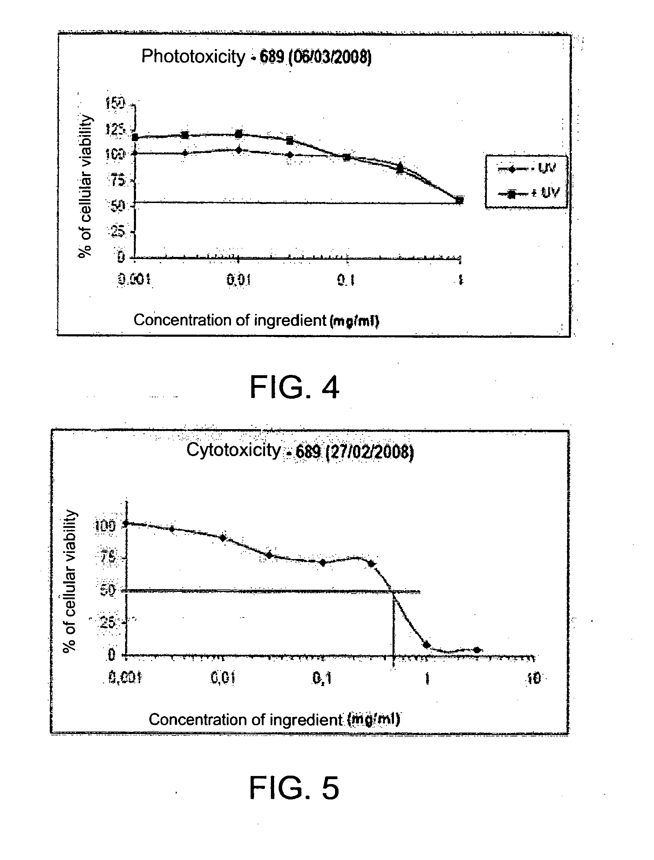

Figure 4 illustrates the result of the test of phototoxicity related to the extract of macela-do-campo.

Figure 5 illustrates the result of the test of cytotoxicity related to the extract of macela-do-campo.

As a result, it was concluded that the extract of macela-do-campo obtained by the process in accordance with the present invention did not present phototoxic potential in any of its tested concentrations and that the cytotoxic potential is low, presenting IC₅₀=0.5 mg/ml. Accordingly, the aforesaid dried extract of macela-do-campo can be indicated for topical use for human beings.

### IV. Inhibition Halo in a culture of Propionibacterium acnes

This trial was performed in order to determine the bacteriostatic potential of the extract of inflorescences of macela-do-campo resulting from the process according to the present invention, or rather, the capacity that the extract has to inhibit the development of the colony of *Propionibacterium acnes.*

For these tests, 0.5% of the dried extract of inflorescences of macela-do-campo was applied in a basic cosmetic formulation (base A).

The measurements of the inhibition halo of the dried extract of macela-do-campo applied to a basic cosmetic formulation were compared to the pharmaceutical product available in the market, BENZAC AC^{®} produced by Galderma, which has benzoyl peroxide 5.0% as an active ingredient.

**Table 2 shows the results obtained in the test:**

| Formulations tested | Measured Halo |
|---|---|
| base A - no extract | Absent |
| base A - 0.10% of extract | Absent |
| base A - 0.25% of extract | Absent |
| base A - 0.50% of extract | 10.0 mm |
| base A - 1.50% of extract | 15.0 mm |
| BENZAC AC^{®} 5.0% | 10.0 mm |

Based on the results listed above it can be observed that the basic cosmetic composition with 0.5% of extract of macela demonstrated the same results as the referenced product BENZAC AC®, despite presenting an active anti-microbial ingredient in a concentration 10 times less in the formula. It is also demonstrated that the cosmetic base for the application of the extract does not present any capacity of inhibition of *Propionibacterium acnes.*

### V. Minimum Inhibitory Concentration (MIC)

Tests were carried out for evaluating the minimum inhibitory concentrations of 3 batches of the dried extract of inflorescences of macela-do-campo produced in accordance with the process of the present invention against *Propionibacterium acnes.*

The procedure used for this test is the following:
Preparation of inoculum: the microorganism to be used in testing was replicated to a glass flask containing 10 ml of sterilized culture medium broth (TSB);
- the flaks were incubated at 35°C + / - 2°C for 24 hours;
- the above procedure was repeated for 2 consecutive days;
- 100 microliters of TSB broth were distributed in a microplate with the exception of column 1;
- column 1 was inoculated with 200 microliters of the sample to be tested previously prepared and solubilized;
- dilutions were carried out by transferring 100 microliters of column 1 to column 2 and 100 microliters of column 2 to column 3 and thus successively until column 10. Columns 11 and 12 were reserved for the inoculum and TSB medium controls respectively;
- after the dilutions were carried out, 20 microliters of each microorganism to be tested were inoculated in all wells of the microplate with the exception of column 12 (control of culture medium);
- the inoculated microplates were incubated with bacteria at 35°C + / - 2°C for 48 hours and the microplates inoculated with yeasts and molds at 25°C + / - 2°C for 72 hours. The plate in which *Propionibacterium acnes* was inoculated was incubated under anaerobosis at 35°C + / - 2°C for 96 hours;
- after the incubation period the reading was carried out, thus observing the turbidity of the wells;
- the samples were weighed and diluted in the vehicle ethanol 96 ° GL / distilled water (1:1) according to the weights and volumes below:

| Sample | quercetin | BDP 009960 | BDP 009960 | BDP 009960 |
|---|---|---|---|---|
| Batch | MM2-7016 | PI001 | PI002 | PI003 |
| Quercetin content | 100.0 | 2.95 | 2.60 | 3.01 |
| Sample mass(mg) | 29.1 | 1005.9 | 1122.2 | 990.9 |
| Initial Dilution (ml) | 100.0 | 100.0 | 100.0 | 100.0 |

The dilution was assisted by microwave for 30 minutes. After dilution the samples were filtered by gravity on quality filter paper. The dilutions were calculated so that the batches of sample BDP 09960 contained the same of concentration of quercetin among themselves and in relation to the concentration of diluted pure quercetin.

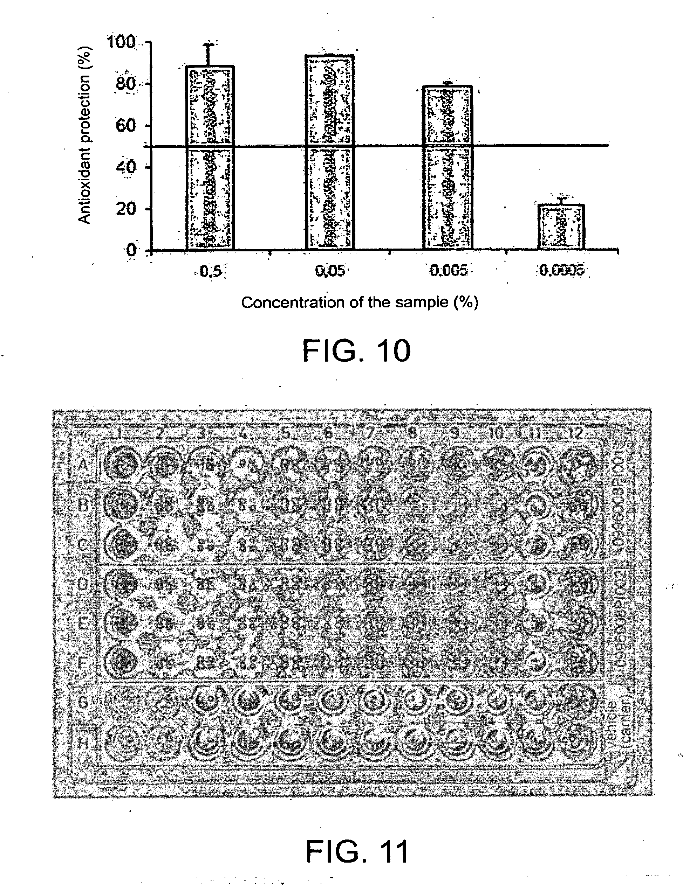

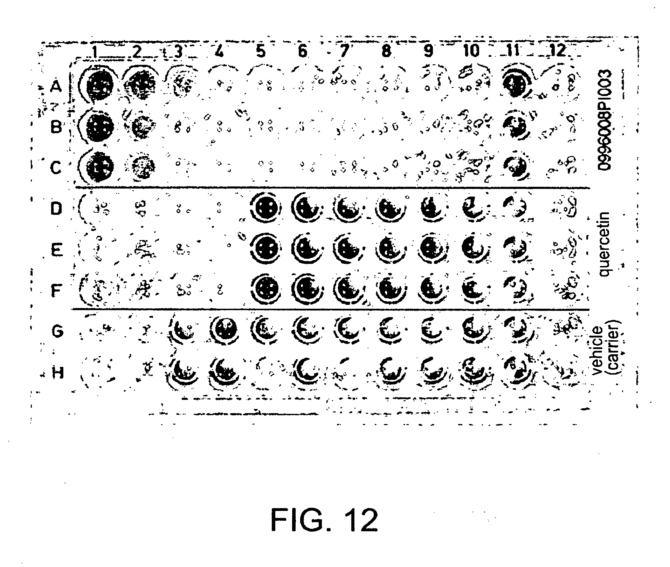

The prepared concentrations failed to determine the MIC, but the results showed that for each batch, named as PI001, PI002 and PI003 MICs fall below 0.020, 0.022 and 0.019 mg / ml, respectively, as illustrated in Figures 11 and 12 and shown in tables below:

**Table 3:**

| | 1 | 2 | 3 | 4 | 5 | 6 | 7 | 8 | 9 | 10 | 11 | 12 |
|---|---|---|---|---|---|---|---|---|---|---|---|---|
| A | 10,059/ 0.2967* | 5,030/ 0.1484* | 2,515/ 0,0742* | 1,257/ 0,0371* | 0,629/ 0,0185* | 0,314/ 0,0093* | 0,157/ 0,0046* | 0,079/ 0.0023* | 0,039/ 0,0012* | 0,020/ 0.0006* | inóculo | TSB |
| B | 10,059/ 0.2967* | 5,030/ 0.1484* | 2,515/ 0.0742* | 1,257/ 0.0371* | 0,629/ 0,0185* | 0,314/ 0,0093 | 0,157/ 0,0046* | 0,079/ 0,0023* | 0,039/ 0,0012* | 0,020/ 0,0006* | inóculo | TSB |
| C | 10,059/ 0,2967* | 5,030/ 0,1484* | 2,515/ 0,0742* | 1,257/ 0.0371* | 0,629/ 0.0185* | 0,314/ 0,0093* | 0,157/ 0.0046* | 0,079/ 0,0023* | 0,039/ 0,0012* | 0,020/ 0,0006* | inóculo | TSB |
| D | 11,222/ 0,2918* | 5,611/ 0,1459* | 2,806/ 0,0729* | 1,403/ 0,0365* | 0,701/ 0,0182* | 0,351/ 0,0091* | 0,175/ 0,0046* | 0,088/ 0,0023* | 0,044/ 0,0011* | 0,022/ 0,0006 | inóculo | TSB |
| E | 11,222/ 0,2918* | 5,611/ 0,1459* | 2,806/ 0,0729* | 1,403/ 0,0385* | 0,701/ 0,0182* | 0,351/ 0,0091* | 0,175/ 0,0046* | 0,088/ 0.0023* | 0,044/ 0,0011* | 0,022/ 0.0006 | inóculo | TSB |
| F | 11,222/ 0,2918* | 5,611/ 0,1459* | 2,806/ 0,0729* | 1,403/ 0,0365* | 0,701/ 0,0182* | 0,351/ 0,0091* | 0,175/ 0,0046* | 0.088/ 0.0023* | 0,044/ 0.0011* | 0,022/ 0,0008 | inóculo | TSB |
| G | veículo | veículo | veícuto | veículo | veículo | veículo | veículo | veículo | veículo | veículo | inóculo | TSB |
| H | veículo | veículo | veículo | veículo | veículo | veículo | veículo | veículo | veículo | veículo | inóculo | TSB |

| | | | | | | | | | | | | |
|---|---|---|---|---|---|---|---|---|---|---|---|---|
| Notes: The concentrations are shown in mg/ml of sample; (*) = concentration calculated as the quercetin content present in the sample TSB = culture medium "Veículo" = vehicle "inoculo" = inoculum | | | | | | | | | | | | |

**Table 4:**

| | 1 | 2 | 3 | 4 | 5 | 6 | 7 | 8 | 9 | 10 | 11 | 12 |
|---|---|---|---|---|---|---|---|---|---|---|---|---|
| A | 9,909 0,2933* | 4,955 0,1491* | 2,477 0,0746* | 1,239 0,0373* | 0,619 0,0186* | 0,310 0,0093* | 0,155 0,0047* | 0,077 0,0023* | 0,039 0,0012* | 0,019 0,0006* | inóculo | TSB |
| B | 9,909 0,2983* | 4,955 0,1491* | 2,477 0,0746* | 1,239 0,0373* | 0,619 0,0186* | 0,310 0,0093* | 0,155 0,0047* | 0,077 0,0023* | 0,039 0.0012* | 0,019 0,0006* | inóculo | TSB |
| C | 9,909 0,2983* | 4,955 0,1491* | 2,477 0,0746* | 1,239 0,0373* | 0,619 0,0186* | 0,310 0,0093* | 0,155 0,0047* | 0,077 0,0023* | 0,039 0,0012* | 0,019 0,0006* | inóculo | TSB |
| D | 0,2910 | 0,1455 | 0,0728 | 0,0364 | 0,0182 | 0,0091 | 0,0045 | 0,0023 | 0.0011 | 0,0006 | inóculo | TSB |
| E | 0,2910 | 0.1455 | 0,0729 | 0,0354 | 0,0182 | 0,0091 | 0,0045 | 0,0023 | 0,0011 | 0,0006 | inóculo | TSB |
| F | 0.2910 | 0,1455 | 0,0728 | 0,0364 | 0,0182 | 0,0091 | 0,0045 | 0,0023 | 0,0011 | 0,0006 | inóculo | TSB |
| G | veículo | veículo | veículo | veículo | veículo | veículo | veículo | veículo | veículo | veículo | inóculo | TSB |
| H | veículo | veículo | veículo | veículo | veículo | veículo | veículo | veículo | veículo | veículo | inóculo | TSB |

| | | | | | | | | | | | | |
|---|---|---|---|---|---|---|---|---|---|---|---|---|
| Notes: The concentrations are shown in mg/ml of sample; (*) = concentration calculated as the quercetin content present in the sample TSB = culture medium "Veículo" = vehicle "inoculo" = inoculum | | | | | | | | | | | | |

The MIC value found for standard quercetin with purity degree of 100% was 0.0364 mg / ml. Since the maximum dilution of the extract of macela-do-campo tested for the three batches is of about 0.002 mg / ml and knowing that the MIC for that extract is a range of values lower than this, it is concluded that the power of that extract for the activity of inhibition of the growth of *Propionibacterium acnes* is at least of the same magnitude of quercetin itself in a state of maximum purity. Additionally, taking into account t the concentration of quercetin batches PI001, PI002 and PI003, namely 2.95, 2.60 and 3.01%, it is noticed that the concentration of quercetin present in the maximum dilution of the macela-do-campo extract of the three batches that still have activity in inhibition of growth of *Propionibacterium acnes* is about 0.0006 mg / ml, that is, a concentration approximately 60 times lower than the MIC of quercetin. Thus, although it was not reached the maximum dilution of said macela-do-campo extract to determine the MIC, it is clear that the activity of growth inhibition of *Propionibacterium acnes in vitro* can not be attributed only to the presence of quercetinno in that extract obtained in accordance with the process of the invention. Other chemicals, such as 3-O-methylquercetin contribute to the overall effect of the extract as far as that activity is concerned.

### VI. Bioautography of Propionibacterium acnes

Bioautographic trials were conducted to determine the classes of the compounds present in the extract of inflorescence of *Achyrocline satureioides* that perform the anti-bacterial activity against *Propionibacterium acnes.* The bioautography consists of the chromatographic separation of the compounds by HPTLC and the subsequent application of the inoculation to the culture medium over the chromatographic plate.

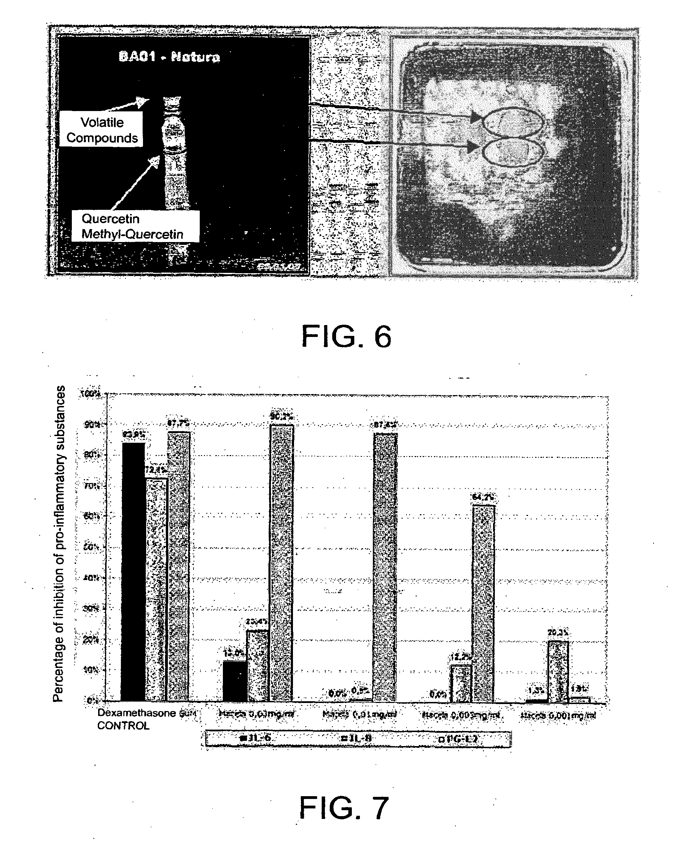

Figure 6 illustrates, on the left, the separation of the compounds of the extract of inflorescence of macela-do-campo with the solvent system of chloroform/ ethyl acetate /methanol/water (60:32:12:8) and revealed with NP/PEG. On the right, that figure shows the inhibition halos formed after the incubation for 72 hours with P. *acnes* ATCC 6538 and revealed with tetrazolium chloride (TTC).

The results obtained in the bioautography trial demonstrate that the flavonoids quercetin and 3-O-methylquercetin are the main ingredients responsible for the anti-microbial activity.

### VII. Anti-inflammatory activity in a model of fibroblasts in an inflammatory model induced by LPS (lipopolysaccharide of a gram-negative bacteria membrane) and by UVB radiation.

The anti-inflammatory potential of the dried extract of inflorescence of macela-do-campo was evaluated by means of the dosage of proinflammatory cytokines, using a culture of human skin fibroblasts. Lipopolysaccharide of a gram-negative bacteria membrane (LPS) or UVB irradiation were used to induce the stimulus for production of cytokines IL-6, IL-8 and of the prostaglandin PG-E2 in human fibroblasts in a culture with different concentrations of extract.

Figure 7 illustrates the result of the test of activity of the extract of inflorescence of macela-do-campo in a model of fibroblasts with induction by LPS.

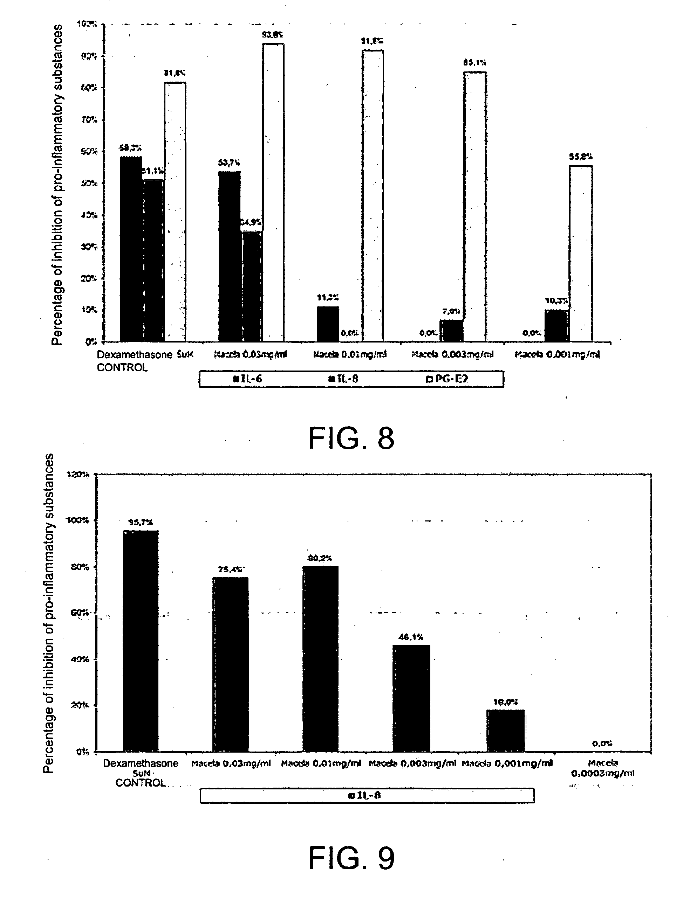

Figure 8 illustrates the result of the test of activity of the extract of inflorescence of macela-do-campo in a model of fibroblasts with induction by UVB.

It is concluded that, in the stimulation model with LPS, the extract presented a significant reduction in the secretion of PG-E2 in concentrations between 0.003 mg/ml and 0.03 mg/ml. In the stimulation model with UVB, a significant reduction was noticed in the secretion of IL-6 and IL-8 in the concentration of 0.03 mg/ml, and inhibition of PG-E2 in concentrations between 0.001 mg/ml and 0.03 mg/ml. In accordance with the analyzed data, the extract of macela-do-campo indicates anti-inflammatory activity in human skin (dermis) fibroblasts *in vitro.*

### VIII. Anti-inflammatory activity in a model in vitro in a culture of human keratinocytes

The anti-inflammatory potential of the dried extract of inflorescences macela-do-campo was evaluated by means of the dosage of proinflammatory cytokines, using a culture of keratinocytes of the HaCat lineage. Inactivated *Propioniumbacterium acnes* was used to induce the production of IL-8 in keratinocytes of the lineage HaCat in a culture with different concentrations of the extract, such as it is commonly achieved with LPS (lipopolysaccharide of a gram-negative bacteria membrane).

Figure 9 illustrates the result of the trial of anti-inflammatory activity of the extract of inflorescence of macela-do-campo in a model of lineage of human keratinocytes (HaCat) with induction by *Propioniumbacterium acnes.*

It is concluded that, in the stimulation model with *P. acnes,* the extract of inflorescences of macela-do-campo obtained in accordance with the process of the present invention presents a reduction greater than 30% in the secretion of II-8 in the concentrations between 0.003 mg/ml and 0.03 mg/ml. In accordance with the analyzed data, the extract of macela-do-campo indicates potential anti-inflammatory activity in human keratinocytes of the lineage HaCat *in vitro.*

### IX. Test of antioxidant activity

Tests were performed for the antioxidant activity by DPPH of the dried extract of macela-do-campo obtained by spray dryer. The tests indicated that the IC50 of the antioxidant activity was obtained with a concentration of dried extract of macela-do-campo of 0.02 mg/ml. Trolox (a derivative of vitamin E sold by Hoffman LaRoche) was used as a standard of the aforesaid test and has its IC50 of antioxidant activity obtained with 0.005 mg/ml. Therefore, it can be concluded that the dried extract of macela-do-campo has extremely high antioxidant effectiveness, only 4 times less than Trolox.

Figure 10 illustrates the result of the test for the antioxidant activity of the extract of macela obtained in accordance with the process of the present invention.

### - Cosmetic and/or pharmaceutical composition comprising of the extract of macela-do-campo:

The cosmetic and/or pharmaceutical composition, also disclosed herein contains a physiologically acceptable carrier and from 0.1% to 5.0%, in mass, preferably, from 0.1 % to 0.5% in mass, based on the total mass of the composition, of extract of inflorescences of macela-do-campo obtained from the process of extraction of inflorescences of *Achyrocline satureioides* in accordance with the present invention.

A cosmetic, pharmaceutical or veterinary composition, disclosed herein, comprising of an extract of Achyrocline satureioides obtained by the process of the present invention is the only one that presents antioxidant, anti-inflammatory and anti-microbial activity due to the high quantities of quercetin and 3-O-methylquercetin.

Due to the presence, in the composition, of the extract of macela-do-campo obtained in accordance with the process of the present invention - containing high concentrations of quercetin and 3-O-methyl-quercetin - the cosmetic and/or pharmaceutical composition disclosed herein presents anti-inflammatory, antioxidant and anti-microbial activity.

The cosmetic and/or pharmaceutical composition may be used in the prevention and treatment of acne, specifically due to its anti-inflammatory and anti-microbial activities against *Propionibacterium acnes* and antioxidant activity. Among the examples of damage arising from inflammatory, microbial and oxidative reactions can be cited: expression wrinkles, signs of cutaneous photoaging, cellulite, acne, skin dyschromia and topical dermatitis, skin (dermis) hypersensitivity, among others.

Also, by presenting high concentrations of these chemical compounds, this composition can also be used for erectile dysfunction and for the pharmacological treatment of bronchial asthma (reference is made to scientific articles listed below).

An ethanolic extract of inflorescences of *Achyrocline satureioides* shows relaxant activity in the smooth muscle of guinea pig *corpus cavemosum.* This activity is primarily due to the molecules of quercetin and 3-O-methylquercetin present in the extract in 0.02% (m/m) and 0.07% (m/m), respectively. These compounds, in a isolated form, show the same relaxant activity (Hnatyszyn et al., 2004). The action mechanism of this activity provided by 3-O-methylquercetin and putatively also by quercetin is the inhibition of the enzyme phosphodiesterase by the subsequent increase of the cAMP and cGMP mediators, with reduction of the concentration of intracellular Ca (Ko, et al. 2001). In another study, it was evidenced that quercetin is a potent inhibitor of PDE 5A, with IC50 of 1.9 µM, while 3-O-methylquercetin is less selective for this enzyme, with IC50 of 86.9µM (Lines & Ono, 2006). The inhibition of the PDE 5A is the same mechanism by which sildenafil assists the vasodilatation, thus assisting the erection in humans suffering from erectile dysfunction. It can be concluded that the quercetin and the 3-O-methylquercetin contained in the extract of *Achyrocline satureioides* are among the molecules responsible for the relaxant activity in the smooth muscle of guinea pig *corpus cavemosum* and because of this there could be an indication for the use of the standardized extract of *Achyrocline satureioides* cited in the present invention in the form of a medication for the pharmacological treatment of erectile dysfunction in humans.

In an experimental model *in vitro* of relaxant muscular activity in guinea pig tracheas, it was shown that 3-O-methylquercetin is capable of provoking the relaxation of the smooth muscle of tracheas that were pre-contracted with histamine, carbachol, KCI and forskolin by the mechanism of the competitive enzymatic inhibition of phosphodiesterase. This inhibition results in the reduction of AMPc and GMPc with the subsequent reduction of intracellular Ca⁺, resulting in the muscular relaxation (Ko et al., 2002). In another study, it was demonstrated that 3-O-methylquercetin is a dual inhibitor of phosphodiesterase 3 and 4 (PDE3/4), more selective for the PDE3 than for the PDE4, with respective IC50 of 1.6µM and 28.5µM. (Chiu& Chang, 1998 apud Ko et al., 2007). The dual inhibitors of PDE 3/4 are more effective in the control of asthma than the simple inhibitors of PDE4. These results indicate that the standardized extract obtained in accordance with this invention, can be indicated for use in pharmaceutical and veterinary products for the pharmacological treatment of bronchial asthma and other pathologies involving bronchial hyperresponsiveness.

The more relevant examples of products that can be prepared from the extract of macela obtained by the process of the present invention or from the cosmetic and pharmaceutical compositions comprising of said extract are:
❖ Moisturizing body milk;
❖ Moisturizing milk for the face;
❖ Moisturizing body lotion;
❖ Moisturizing lotion for the face;
❖ Gels;
❖ Products for the scalp;
❖ Sun protectors or blockers for adult or child use, destined or not destined for the concomitant use in the practice of sports;
❖ Body or facial moisturizers;
❖ Body or facial anti-blemish (skin care) products;
❖ Body or facial firming products;
❖ Self tanning products;
❖ Insect repellent products;
❖ Body or facial moisturizing skin illuminators;
❖ Pharmaceutical preparations for topical application;
❖ Body or facial cosmetic preparations for child use;
❖ Localized action cosmetic preparations, specifically for the periocular region, the contour of the lips, the lips, anti-blemish and anti-dark circles under the eyes, among others;
❖ Anti-acne products;
❖ Products for skin lightening;
❖ Pharmaceutical compositions for the treatment of specific dermatoses;
❖ Lipsticks and wax bases;
❖ Blushes and pigmentation foundations;
❖ Facial powders;
❖ Any make-up product for the area of the eyes;
❖ Anti-cellulite products;
❖ Products for sensitive skin;
❖ Tonics;
❖ Dentifrices;
❖ Mouth wash.

### - Components of the composition:

### Extract of macela-do-campo:

The extract of macela-do-campo is obtained by an extraction process of inflorescences of *Achyrocline satureioides* in accordance with the process of the present invention as described above.

### Carrier

Water is the basis of several preferred cosmetic compositions disclosed herein, acting as the carrier for the other components. The compositions disclosed herein comprise water, preferentially demineralized or distilled, in an appropriate percentage (q.s.) to achieve 100% of the formula based on the total weight of the present composition. Naturally, other cosmetically acceptable carriers can be used.

### Optional components:

Some examples of cosmetic adjuvants compatible with the properties of the composition described herein will next be listed below - in a non-restrictive manner, only for demonstrative purposes - which, additionally, can be employed in the present cosmetic and/or pharmaceutical composition destined for the prevention and treatment of damage arising from inflammatory, microbial and oxidative/lipoperoxidative reactions:
- Antioxidant agents: BHT, tocopherol and/or its derivatives, catechols, tannins and/or its derivatives, phenolic compounds and hydroquinone, among others;
- Preservative agents: methylparabens, propylparabens, isothiazolinones, phenoxyethanol;
- Film forming agents: agar gum, carrageenan gum, alginates, gum arabic, gelatin;
- Forming agents for a sustentation micro-crystalline network: dextrans, methyl-acrylates, PHB, PHA;
- Polymeric agents and/or co-polymeric agents: copolymers of silicone, polymers of siloxane and/or modified silicone, co-polymers of acrylate;
- Denaturing agents: denatonium benzoate;
- Consistency agents: plant waxes, mineral hydrocarbonates, paraffin, beeswax, white wax, whale spermaceti, cocoa butter, shea butter, sugar cane wax;
- emollients: liquid paraffin, palm oil, cupuaçu butter, lecithin, milk amino acids, wheat protein, vegetable proteins, phospholipid vegetable oils, ceramides, ceramides of passion fruit, sphingolipids, whale spermaceti, lanoline, almond oil, dicapryl carbonate, silicone elastomers, cyclomethicone;
- Humidifying agents and/or moisturizing agents: glycerin, propylenoglycol, hyaluronic acid, urea, PCA;
- Conditioning agents: quaternary ammonium salts, silicones, siloxanes; and
- Assets.

The present cosmetic and/or pharmaceutical composition presents other physical-chemical characteristics that are appropriate and safe for the user of the end product:
1. It is stable for a period of at least two years;
2. It has an appropriate texture during application that is non-stick and non-oily;
3. It is easily spread;
4. It does not make the skin oily after application;
5. It does not present comedogenicity;
6. It does not present phototoxicity and cytotoxicity;
7. It does not present allergenicity;
8. It is not sensitizing;
9. It does not provoke any type of adverse reaction or cutaneous or ocular lesion, whether in normal conditions of use or in conditions of forced perspiration;
10. It presents an excellent level of homogeneity and stability;
11. By not provoking irritation to the skin, it becomes more comfortable and allows for daily use or for even more than once per day;
12. It presents appropriate chemical stability;
13. It comprises of a multifunctional extract of macela that presents anti-microbial, antioxidant and anti-inflammatory activities. It also presents low cost in the preparation of the composition as it dispenses with the addition of complex antioxidants and preservatives;
14. The extract of macela presents a pleasant color and smell that do not impact upon the final result of the composition;
15. The extract presents sensorial characteristics (softness, texture and shine) that are ideal for cosmetic compositions of topical use;
16. The extract combats the specific inflammation induced by the chemical components present in the membrane of the *Propionibacterium acnes.*

### Example of a cosmetic and/or pharmaceutical composition comprising of an extract of macela-do-campo:

The following examples are preferential embodiments of cosmetic and/or pharmaceutical compositions destined for the prevention and treatment of the damage arising from inflammatory, microbial and oxidative reactions and should not, therefore, be interpreted as limitations of the same. In this context, it should be understood that the scope of the present invention has other possible embodiments and is limited by the content of the attached claims, including all the possible equivalents therein.

In table 5 an example is described of one embodiment (formulation) of the cosmetic and /or pharmaceutical composition, object of the present invention:

### - Example 1: Astringent lotion with macela

**Table 5:**

| Phase | Component | Quantity (% in mass) |
|---|---|---|
| 1 | Vegetable bidistilled glycerin | 2.00 |
| 1 | Demineralized water | Qs |
| 1 | Glycereth-26 | 3.00 |
| 2 | Zinc pca | 0.75 |
| 3 | Salicylic acid | 0.50 |
| 3 | Demineralized water | 5.00 |
| 3 | Extract of macela do campo | 0.10 |
| 3 | Organic hydrated neutral ethyl alcohol | 20.00 |
| 3 | Denatonium benzoate solution 20% p/v | 0.0006 |
| 4 | Hydrogenated castor oil | 2.00 |
| 5 | Tocopheril acetate | 0.25 |
| 5 | Fraqrance | 0.10 |
| 5 | Certified natural alpha bisabolol | 0.50 |
| 5 | Methyl-4-isothiazolin-3-one | 0.07 |
| 5 | Phenoxyethanol | 0.40 |
| 6 | Demineralized water | 3.00 |
| 6 | Sodium hydroxide | 0.05 |

### Preparation of the cosmetic composition of example 1

a. homogenize the components of phase 1;
b. add to the phase obtained in (a) the components of phase 2 and homogenize;
c. add to the phase obtained in (b) the previously solubilized components of phase 3 and homogenize;
d. heat the components of phase 4 until they are completely solubilized and then cool;
e. when the temperature is less than 40°C, solubilize the components of phase 5 over phase 4 and add to the principal phase (obtained in (c)) and homogenize;
f. add the previously solubilized components of phase 6 in water to the phase obtained in (e) and homogenize.

### BIBLIOGRAPHICAL REFERENCES

CARNEY, J.R. et al. Achyrofuran, a new antihyperglycemic dibenzofuran from the South American medicinal plant Achyrocline satureioides. J. Nat. Prod., 65(2): 203-205, 2002.
CHAN AL, Huang HL, Chien HC, Chen CM, Lin CN, Ko WC. Inhibitory effects of quercetin derivatives on phosphodiesterase isozymes and high-affinity [(3) H]-rolipram binding in guinea pig tissues. Invest New Drugs. Oct;26(5):417-24, 2008,
D'ÁVILA, M.C. Da flora medicinal do Rio Grande do Sul. Dissertação Faculdade Livre de Medicina e Farmácia de Porto Alegre, 1910. Porto Alegre. 155 pp. (Medicinal flora of Rio Grande do Sul. Dissertation Faculty of Medicine and Pharmacy of Porto Alegre*)*
HNATYSZYN, O. et al. Flavonoids from Achyrocline satureioides with relaxant effects on the smooth muscle of Guinea pig corpus cavernosum. Phytomedicine, 11(4): 366-369, 2004.
IKUTA, A.R.Y. Estudos sobre Propagação de marcela Achyrocline satureioides (Lam.) (Studies on the propagation of marcela, Achyrocline satu-reioides (Lam.)) D.C., Composite. 205 p. Dissertation (Master's Degree in Plant Science) - Master's Course in Plant Science, Federal University of Rio Grande do Sul, Porto Alegre, 1993.
JIANG, J.-S. et al. Potent suppressive effects of 3-O-methylquercetin 5,7,3',4'-O-tetraacetate on ovalbumin-induced airway hyperresponsiveness. Planta Medica 73(11):156-1162, 2007.
KADARIAN, C. et al. Hepatoprotective activity of Achyrocline satureioides (Lam) D. C. Pharmacol. Res., 45(1): 57-61, 2002.
KO WC, Wang HL, Lei CB, Shih CH, Chung MI, Lin CN. Mechanisms of relaxant action of 3-O-methylquercetin in isolated guinea pig trachea. Planta Med. Jan;68(1):30-5, 2002
LEMOS, G.C.S. Bacterial activity of macela (Achyrocline satureioides) against strains of Staphylococcus aureus isolated from subclinical bovine mastitis Rev. Bras. Pl. Med., 3(1): 67-72, 2000.
LINES, T.C.; ONO, M. FRS 1000, an extract of red onion peel, strongly inhibits phosphodiesterase 5a (pde 5a). PHYTOMEDICINE, v.13, n.4, p. 236-239,2006.
MARQUES, F.C. & Barros, I.B.I. Crescimento inicial de marcela (Achyrocline satureioides) em ambiente protegido (Initial growth of marcela (Achyrocline satureioides) in a protected environment). Rural Science, 31(1): 517-518, 2001.
MARQUES, F.C. & Barros, I.B.I. Qualidade de sementes de marcela (Achyrocline satureioides) provenientes de duas populações do Rio Grande do Sul (Quality of seeds of marcela (Achyrocline satureioides) from two populations of Rio Grande do Sul). Rural Science, 30(2): 241-247, 2000.
MARQUES, F.C. Análise da qualidade de sementes de marcela (Achyrocline satureioides) (Lam.) D.C. (Asteraceae)provenientes de duas populações do Rio Grande do Sul (Analysis of the quality of seeds of marcela, Achyrocline satureioides (Lam.) D. C. (Asteraceae), from two populations of Rio Grande do Su)l. 143 p. Dissertation (Master's Degree in Plant Science) - Master's Course in Plant Science, Federal University of Rio Grande do Sul, Porto Alegre, 1995.
MEDEIROS, M. F.T.; et al Regina Helena P. Flora medicinal dos sitiantes da Reserva Particular do Patrimônio Natural das Pedras, Mangaratiba, Rio de Janeiro, Brasil:taxonomia e aspectos etnobotânicos (Medicinal flora of the farms of the Private Reserve of Natural Heritage, Rio das Pedras, Mangaratiba, Rio de Janeiro, Brazil: Taxonomy and ethnobotanic aspects.) Publ. A-vul. Mus. Nac., Rio de Janeiro, n.106, p. 3-24, Mar. 2005.
OLIVEIRA, A.L. et al. Achyrocline satureioides, Asteraceae, comparative evaluation of the plant ingredient and preliminary studies of the optimization of extraction. Pharmacy Notebook, 17(1): 33-38, 2001.
PARDO, V.A. Estaquia de marcela Achyrocline satureioides (Lam.) D.C. sob diferentes períodos de enraizamento e doses de ácido indolbutírico (Staking of marcela Achyrocline satureioides (Lam.) D.C. under different periods of root planting and doses of indolebutyric acid). 78p. Dissertation (Master's Degree in Plant Science) - Master's Course in Plant Science, Federal University of Rio Grande do Sul, Porto Alegre,1995.
RITTER et. al. Plantas usadas como medicinais no municipio de Ipê. (Plants used as medicines in the municipality of Ipê), RS. Rev. Bras. Farmacogn., 12(2): 51-62, 2002.
SIMÕES, C.M.O. Antiinflammatory action of Achyrocline satureioides extracts applied topically. Fitoterapia, 59(5): 419-421, 1988.

## Claims

1. An extraction process for obtaining a standardized extract of quercetin and 3-O-methylquercetin from inflorescences of macela-do-campo *(Achyrocline satureioides)* **characterized by** comprising of the following steps:
A) grinding the inflorescences of *Achyrocline satureioides* to obtain a material of ground plant;
B) submitting the ground plant to at least four sequential stages of hydro-alcoholic extraction at a temperature from 60°C to 80°C, for 3 to 4 hours for each stage, in order to obtain 4 intermediary hydro-alcoholic extracts;
C) combining the 4 intermediary hydro-alcoholic extracts;
D) concentrating the intermediary hydro-alcoholic extracts mixture; in order to obtain up to a maximum of 20% of the initial mass of the intermediary hydro-alcoholic extracts;
E) drying the material obtained in (D).

2. Process, according to claim 1, **characterized in that** in step B the sequential stages of the hydro-alcoholic extraction are carried out under reflux, for 12 hours and comprise the following stages:
i) extracting the intermediary extract with a hydro-alcoholic solution 1:30 (m/m of ground plant material/extracting solution) for a period of 3 to 4 hours, preferably for at least 3 hours, at a temperature in the range of 60 to 80°C, preferably at 80 °C, in order to obtain a first extracted solution;
ii) re-extracting the hydro-alcoholic extract of (i) with a hydro-alcoholic solution in the proportion of 1:20 (m/m of plant drug/extracting solution) for a period of 3 hours at a temperature of 80 °C thus obtaining a second extracted solution;
iii) combining the first and the second extracted solutions in order to obtain a third extracted solution;
iv) extracting a fresh batch of ground plant with said third extracted solution in the proportion of 1:30 (m/m of ground plant ingredient/extracted solution of stages 1 and 2) for a period of 3 hours at a temperature of 80 °C ;
v) re-extracting the hydro-alcoholic extract from stage (iv) with a hydro-alcoholic solution in the proportion of 1:16 (m/m of hydro-alcoholic extract /extracting solution) for 3 hours at 80 °C in order to obtain a final extracted solution.

3. Process according to claim 1, **characterized in that** step C is carried out in a vacuum concentrator at temperature of 60°C and a pressure of 600 mmHg.

4. Process according to claim 1, **characterized in that** said final extracted solution is 5 times more concentrated than the initial solution.

5. Process according to claim 1, **characterized in that** in step D an appropriate carrier for spraying is added in a proportion of 50% by mass and the subsequent drying is carried out in a spray dryer.

## Patentansprüche

1. Extraktionsverfahren zum Erhalten eines standardisierten Extrakts von Quercetin und 3-O-Methylquercetin aus Blüten von Macela-do-campo (*Achyrocline satureioides*), **dadurch gekennzeichnet, dass** es die folgenden Schritte umfasst:
A) Vermahlen der Blüten von *Achyrocline satureioides,* um ein Material aus vermahlener Pflanze zu erhalten;
B) Unterwerfen der vermahlenen Pflanze mindestens vier aufeinander folgenden Stufen von wässrig-alkoholischer Extraktion bei einer Temperatur von 60°C bis 80°C, 3 bis 4 Stunden lang für jede Stufe, um 4 zwischengeschaltete wässrig-alkoholische Extrakte zu erhalten;
C) Vereinigen der 4 zwischengeschalteten wässrig-alkoholischen Extrakte;
D) Aufkonzentrieren der Mischung der wässrig-alkoholischen Extrakte, um bis zu maximal 20% der Ausgangsmasse der wässrig-alkoholischen Extrakte zu erhalten;
E) Trocknen des in (D) erhaltenen Materials.

2. Verfahren gemäß Anspruch 1, **dadurch gekennzeichnet dass** bei dem Schritt B) die aufeinander folgenden Stufen von wässrig-alkoholischer Extraktion 12 Stunden lang unter Rückfluss durchgeführt werden und die folgenden Stufen umfassen:
i) Extrahieren des zwischengeschalteten Extraktes mit einer wässrigalkoholischen Lösung 1:30 (m/m des vermahlenen Pflanzenmaterials/Extraktionslösung) über einen Zeitraum von 3 bis 4 Stunden, vorzugsweise mindestens 3 Stunden, bei einer Temperatur im Bereich von 60 bis 80°C, vorzugsweise bei 80°C, um eine erste extrahierte Lösung zu erhalten;
ii) erneutes Extrahieren des wässrig-alkoholischen Extraktes aus (i) mit einer wässrig-alkoholischen Lösung im Verhältnis von 1:20 (m/m der Pflanzendroge/Extraktionslösung) über einen Zeitraum von 3 Stunden bei einer Temperatur von 80°C, um auf diese Weise eine zweite extrahierte Lösung zu erhalten;
iii) Vereinigen der ersten und der zweiten extrahierten Lösungen, um eine dritte extrahierte Lösung zu erhalten;
iv) Extrahieren eines frischen Ansatzes von vermahlener Pflanze mit der dritten extrahierten Lösung im Verhältnis von 1:30 (m/m des vermahlenen Pflanzeninhaltsstoffes/extrahierte Lösung der Stufen 1 und 2) über einen Zeitraum von 3 Stunden bei einer Temperatur von 80°C;
v) erneutes Extrahieren des wässrig-alkoholischen Extraktes aus der Stufe (iv) mit einer wässrig-alkoholischen Lösung im Verhältnis von 1:16 (m/m des wässrig-alkoholischen Extrakts/Extraktionslösung) 3 Stunden lang bei 80°C, um eine endgültige extrahierte Lösung zu erhalten.

3. Verfahren gemäß Anspruch 1, **dadurch gekennzeichnet dass** der Schritt C in einem Vakuum-Konzentrator bei einer Temperatur von 60°C und einem Druck von 600 mm Hg durchgeführt wird.

4. Verfahren gemäß Anspruch 1, **dadurch gekennzeichnet dass** die endgültige extrahierte Lösung 5 mal konzentrierter ist als die Ausgangslösung.

5. Verfahren gemäß Anspruch 1, **dadurch gekennzeichnet dass** in Schritt D ein zum Sprühen geeigneter Träger in einem Massenverhältnis von 50% zugesetzt wird und die nachfolgende Trocknung in einem Sprühtrockner durchgeführt wird.

## Revendications

1. Procédé d'extraction permettant l'obtention d'un extrait standardisé de quercétine et de 3-O-méthylquercétine à partir d'inflorescences de macela-docampo *(Achyrocline satureioides),* **caractérisé en ce qu'**il comprend les étapes suivantes :
A) le broyage des inflorescences de *Achyrocline satureioides* pour obtenir une matière de plante broyée ;
B) la soumission de la plante broyée à au moins quatre étapes séquentielles d'extraction hydroalcoolique à une température de 60°C à 80°C, pendant 3 à 4 heures pour chaque étape, afin d'obtenir 4 extraits hydro-alcooliques intermédiaires ;
C) la combinaison des 4 extraits hydro-alcooliques intermédiaires ;
D) la concentration du mélange d'extraits hydroalcooliques intermédiaires afin d'obtenir jusqu'à un maximum de 20 % de masse initiale d'extraits hydro-alcooliques intermédiaires ;
E) le séchage de la matière obtenue au (D).

2. Procédé selon la revendication 1, **caractérisé en ce que**, à l'étape B, les étapes séquentielles d'extraction hydroalcoolique sont réalisées au reflux, pendant 12 heures et comprennent les étapes suivantes :
i) l'extraction de l'extrait intermédiaire avec une solution hydro-alcoolique 1/30 (m/m de matière de plante broyée/solution d'extraction) pendant une durée de 3 à 4 heures, de préférence d'au moins 3 heures, à une température dans la plage de 60 à 80°C, de préférence à 80°C, afin d'obtenir une première solution extraite ;
ii) la ré-extraction de l'extrait hydroalcoolique du (i) avec une solution hydro-alcoolique en la proportion de 1/20 (m/m de drogue végétale/solution d'extraction) pendant une durée de 3 heures à une température de 80°C, ce qui permet ainsi d'obtenir une seconde solution extraite ;
iii) la combinaison des première et deuxième solutions extraites afin d'obtenir une troisième solution extraite ;
iv) l'extraction d'un lot frais de plante broyée avec ladite troisième solution extraite en la proportion de 1/30 (m/m d'ingrédient de plante broyée/solution extraite des étapes 1 et 2) pendant une durée de 3 heures à une température de 80°C ;
v) la ré-extraction de l'extrait hydro-alcoolique de l'étape (iv) avec une solution hydro-alcoolique en la proportion de 1/16 (m/m de l'extrait hydro-alcoolique / solution d'extraction) pendant 3 heures à 80°C afin d'obtenir une solution extraite finale.

3. Procédé selon la revendication 1, **caractérisé en ce que** l'étape C est réalisée dans un concentrateur sous vide à une température de 60°C et à une pression de 600 mmHg.

4. Procédé selon la revendication 1, **caractérisé en ce que** ladite solution extraite finale est 5 fois plus concentrée que la solution initiale.

5. Procédé selon la revendication 1, **caractérisé en ce que**, à l'étape D, un véhicule approprié pour la pulvérisation est ajouté en une proportion de 50 % en poids et le séchage ultérieur est réalisé dans un séchoir de pulvérisation.
